Europäisches Patentamt

European Patent Office

Office européen des brevets

(11) Numéro de publication: **0 277 941 B1**

# FASCICULE DE BREVET EUROPEEN

(45) Date de publication de fascicule du brevet:
**15.01.92**

(51) Int. Cl.⁵: **A61B 5/103**, A61D 17/00

(21) Numéro de dépôt: **86904750.6**

(22) Date de dépôt: **03.09.86**

(86) Numéro de dépôt internationale :
**PCT/BE86/00027**

(87) Numéro de publication internationale :
**WO 87/01573 (26.03.87 87/07)**

(54) **PROCEDE ET DISPOSITIF DE SURVEILLANCE DESTINES NOTAMMENT A LA PARTURITION ET LEUR APPLICATION.**

(30) Priorité: **11.09.85 LU 86072**

(43) Date de publication de la demande:
**17.08.88 Bulletin 88/33**

(45) Mention de la délivrance du brevet:
**15.01.92 Bulletin 92/03**

(84) Etats contractants désignés:
**AT BE CH DE FR GB IT LI LU NL SE**

(56) Documents cités:
**EP-A-00 835 68**
**WO-A-80/00054**
**FR-A- 2 170 007**
**US-A- 3 520 294**
**US-A- 3 989 034**

**Proceedings of the IEEE, vol. 65, no. 5, May 1977, (New York, US) W.C. Lin et al.: "A micro-processor-based data acquisition and processing system for studying the kenematics of labor", pages 722-729**

(73) Titulaire: **AGRITRONICS INTERNATIONAL, Société Anonyme**
**2 rue du Warichet**
**B-5854 Meux-la-Bruyère(BE)**

(72) Inventeur: **COBBEN, Andrée**
**8, rue de Liernu**
**B-5854 Meux-La-Bruyère(BE)**
Inventeur: **JASPAR, Francis**
**8, rue de Liernu**
**B-5854 Meux-La-Bruyère(BE)**
Inventeur: **MOUTHUY, Christian**
**Avenue Victor Hugo 172**
**B-1410 Waterloo(BE)**

(74) Mandataire: **Van Malderen, Michel et al**
**p.a. Freylinger & Associés 22 avenue J.S. Bach (bte 43)**
**B-1080 Bruxelles(BE)**

Rank Xerox (UK) Business Services

## Description

OBJET DE L'INVENTION

La présente invention est relative à un procédé de surveillance fiable de la parturition chez les animaux ou les êtres humains. Elle concerne également un dispositif pour la mise en oeuvre de ladite surveillance et l'application de ce procédé et de ce dispositif en médecine humaine ou vétérinaire ou pour l'élevage.

L'invention sera décrite ci-après essentiellement en relation avec la parturition d'animaux mais ceci n'exclut pas, avec des adaptations éventuelles, l'utilisation pour les êtres humains.

Le développement des races animales de plus en plus performantes va en général de pair avec la nécessité d'intervention extérieure lors de la parturition. Ceci implique la nécessité pour l'éleveur de surveiller son animal de manière tellement soutenue qu'il s'assure quasiment la certitude qu'il sera présent lors de la mise-bas afin d'intervenir lui-même ou de faire intervenir un vétérinaire, au moment opportun. Ne pas être présent à ce moment critique entraîne dans certains cas la mort du nouveau-né et parfois même celle de la mère. Les conséquences économiques de ce phénomène sont évidemment très lourdes pour l'éleveur.

En obstétrique humaine, des moyens matériels et humains plus importants sont mis en oeuvre; néanmoins un dispositif permettant de suivre de manière objective le processus d'accouchement conserve un grand intérêt potentiel, éventuellement en complément à d'autres techniques telles que l'échographie.

RÉSUMÉ DE L'ÉTAT DE LA TECHNIQUE

Dans la grande majorité des cas, les éleveurs surveillent leurs animaux de manière subjective et prennent en compte des éléments d'observation tels par exemple le degré de nervosité, la forme du bassin, la position de la queue et.... pour évaluer si l'animal a des chances de mettre bas dans les quelques heures qui suivent. Cet examen sera répété de très nombreuses fois, de jour et de nuit, au cours des quelques jours qui précèdent la mise-bas.

Il existe néanmoins déjà des systèmes de surveillance permettant de compléter la surveillance purement subjective.

Un système décrit dans le document FR-A-2 413 875 permet de détecter un moment critique, celui de l'apparition des eaux lors de la rupture de la poche des eaux dans le processus de mise-bas.

Ce dernier système a l'avantage de surveiller l'animal de manière objective. Il souffre cependant de deux inconvenients majeurs :

(1) l'appareil de détection est intra-vaginal et présente de cette façon pour l'animal un inconfort et un risque d'infections et pour l'éleveur une difficulté d'utilisation;

(2) l'appareil n'a pas une fiabilité totale car il est connu des spécialistes que bon nombre de parturitions animales se déroulent sans qu'il n'y ait un écoulement significatif des eaux avant la naissance ou sans que l'écoulement ne soit détecté par le système.

D'autres techniques invasives, c'est-à-dire à action intra-vaginale ont été décrites notamment dans BE-A-852 331, BE-A-873 572, BE-A-892 016, FR-A-2 218 870, FR-A-2 266 489, FR-A-2 413 875 et FR-A-2 565 097, EP-A-0 108 330 et DE-A-24 16 829.

Plusieurs techniques non invasives ont également été décrites qui reposent sur l'observation de phénomènes connexes à la parturition.

Certains d'entre-eux sont basés sur la position adoptée par l'animal. C'est le cas par exemple de DE-C-27 12 854, WO-A-82/00952, BE-A-830 865, US-A-4 055 839, NL-B-7906774.

Une autre technique consiste à détecter la présence du jeune après expulsion (FR-A-2 241 239) ou cette expulsion elle-même (US-A-4 232 686, FR-A-2 349 318, FR-A-2 353 225, FR-A-2 514 635, GB-A-632 827, BE-A-864477).

Finalement FR-A-2 389 368 mesure l'humidité de sudation lors de la mise-bas et BE-A-867 412 l'horizontalité de la queue de l'animal pendant un temps supérieur à la normale.

En obstétrique humaine le document US-A-4 299 233 décrit la mesure des vibrations du corps par détermination des ondes sonores dans un matelas ou coussin rempli d'un liquide. Il existe bien entendu également des dispositifs d'échographie pour suivre les accouchements.

Le document US-A-3 520 294 (FUZZEL et al.), décrit la détection de l'activité musculaire abdominale à l'aide d'une ceinture comportant au moins un élément sensible agissant dans le sens longitudinal de déformation de ladite ceinture. Ce document décrit également un circuit électronique de correction automatique de la ligne de base relative à l'activité musculaire.

Le document "Proceedings of the IEE", vol. 65, no. 5, May 1977, pages 722-729 décrit un circuit électronique permettant de suivre l'évolution en amplitude et en fréquence des contractions utérines et abdominales et permettant en fonction de l'évolution de déclencher une alarme ou un système de prévention.

Par le document US-A-3 989 034, on connaît un appareil et procédé de surveillance de l'activité cardiaque et de surveillance de la partirution. Ledit procédé consiste à détecter et à surveiller un signal comprenant une information relative à l'activité musculaire abdominale et en particulier une information relative aux contractions utérines et abdominales et à suivre l'évolution de ce signal en amplitude et en fréquence. Le circuit électronique permet de suivre l'évolution en amplitude et en fréquence et permet ensuite, en fonction de l'évolution, de déclencher une alarme ou un système de prévention.

Le document WO 80/00054 décrit un dispositif de surveillance de la partirution comprenant une ceinture avec un élément sensible pour capter les mouvements abdominaux et des éléments sensibles aux paramètres tels que le rythme cardiaque, la température du corps, le rythme respiratoire. Il fait notamment appel à une jauge de contrainte. Voir aussi le document EP-A2-0 083 568. En outre, le document FR-A-2 170 007 mentionne que l'élément sensible de la ceinture peut également être constitué par une chambre déformable comprenant un fluide tel que l'air et un capteur de pression.

Toutes ces techniques présentent une série d'inconvénients qui sont d'ailleurs décrits pour une grande part dans les documents cités eux-mêmes.

On peut résumer ces inconvénients comme étant leur faible fiabilité, leur mise en place laborieuse, le risque de fausses alertes résultant de mouvements de l'animal, l'inconfort pour l'animal avec pour conséquence des tentatives de se débarasser du dispositif utilisé par frottement par exemple et pour certains d'entre-eux leur prix de revient élevé.

Finalement la plupart des dispositifs connus sont difficilement transposables d'une espèce animale à l'autre et nécessitent même souvent une adaptation spécifique pour chaque variété animale, voire pour chaque animal particulier.

## BUT DE L'INVENTION

En conséquence de ce qui précède et pour des raisons de rendement économique lié à une réduction de pertes d'animaux par décès, par blessure ou par tout autre dévalorisation et à une diminution de la main d'oeuvre nécessaire à la surveillance subjective, il existe une demande importante d'un procédé et d'un dispositif de surveillance fiable de la parturition pour les animaux. Pour l'espèce humaine, toute technique pouvant être utilisée en complément aux moyens connus est bien entendu hautement souhaitable et présente un intérêt évident.

L'invention vise à fournir un tel procédé et dispositif qui évite largement les inconvénients majeurs des solutions de l'état de la technique.

## ÉLÉMENTS CARACTÉRISTIQUES DE L'INVENTION

La solution apportée par l'invention repose sur l'observation que l'activité musculaire au niveau abdominal due directement ou indirectement aux contractions constitue un des signes les plus significatifs du processus de parturition.

La présente invention propose un dispositif de surveillance de la parturition pour les animaux et êtres humains comportant une ceinture de surveillance comportant au moins un élément sensible qui permet de détecter l'activité musculaire abdominale et qui est relié à un circuit électronique de traitement qui permet de suivre l'évolution en amplitude et en fréquence des contractions utérines et abdominales et permet notamment une correction de la ligne de base, caractérisé en ce que le circuit électronique comprend, pour ce faire, un amplificateur des signaux d'entrée, monté en amplificateur différentiel, un conduit du signal des éléments sensibles et une tension de référence étant connectés à une première entrée de l'amplificateur différentiel et un autre conduit du signal des éléments sensibles et une tension de correction de la ligne de base étant connectés à la deuxième entrée de l'amplificateur différentiel, la tension de correction de la ligne de base étant fournie par un microprocesseur après analyse par celui-ci et conversion digitale/analogique du signal de la sortie de l'amplificateur différentiel, ledit microprocesseur faisant également une analyse de l'évolution de l'activité musculaire pour déclencher une alarme ou un système de prévention.

Une difficulté particulière de ce type de ceinture peut être illustrée par l'exemple suivant. En supposant qu'une telle ceinture instrumentée a été mise en place, sur un animal en position debout, si celui-ci se couche, la déformation naturelle du corps dans cette nouvelle position résultera en un signal résultant d'une tension dans la ceinture. Ce signal devra être différencié des signaux d'alerte résultant des contractions utérines et abdominales. À cet effet le dispositif est équipé selon une caractéristique d'une unité de traitement électronique adéquate de signaux dont les détails seront décrits ci-après.

L'unité de traitement électronique permet par une technique de mémorisation de ligne de base, d'identifier à tout instant et lors de toute nouvelle sollicitation s il s'agit d'un signal présentant un spectre nouveau et dont l'allure générale est celle d'une contraction utérine et abdominale. L'unité pourra dès l'instant où cette identification est réalisée mémoriser la contraction et ses paramètres et déclencher une alarme, notamment le système de prévention de l'éleveur, dans le cas où les contraction détectées ont évolué dans leur nombre et/ou leur fréquence et/ou leur amplitude.

L'élément sensible de la ceinture de surveillance peut être une feuille plastique piézo-électrique.

Le phénomène piézo-électrique, c'est-à-dire la création de charges électriques sous l'effet d'une déformation mécanique et vice versa est connu depuis de nombreuses décennies dans des matériaux inorganiques tels que par exemple le quartz ou des céramiques comportant des oxydes de plomb, de zirconium, de baryum, de titane, et....

Ces matériaux sont en général onéreux, fragiles et n'existent qu'en petites surfaces ce qui implique de les manipuler avec précaution quand ils sont utilisés comme détecteurs.

Depuis peu, certains laboratoires ont développé des matériaux organiques piézo-électriques dont le plus connu est le PVDF (difluorure de polyvinyle). Il en existe cependant d'autres, et en particulier des composites comprenant des particules céramiques piézo-électriques enrobées dans une matrice polymérique.

Il est avantageux pour l'application qui fait l'objet de l'invention, d'utiliser un élément piézo-électrique plastique et ce pour les raisons suivantes :

(1) élasticité : comme l'élément piézo-électrique peut être une feuille aussi mince que 10 à 20 microns, elle présente une souplesse telle que même en grande surface, elle épousera parfaitement la forme de l'animal;

(2) grande surface : le fait que l'élément sensible peut être choisi de grande surface (par exemple une bande de 5 à 10 cm de large sur tout le flanc du sujet) entraîne que la mise en place de la ceinture pour son fonctionnement est aisée;

(3) robustesse : un film piézo-électrique assure une grande résistance aux chocs et impacts consécutifs à la mise en place de la ceinture ou lors de son utilisation (frottements de l'animal contre les murs, poutres etc... se trouvant dans son voisinage).

La feuille piézo-électrique est rigidement fixée à ses extrémités à la ceinture qui en assure son support à l'aide d'adhésifs ou encore par d'autres moyens d'ancrage mécaniques.

La ceinture sera avantageusement constituée d'une matière fibreuse synthétique (par exemple en polyester) assurant une certaine élasticité mais elle pourrait comprendre aussi une partie non déformable et une partie élastique.

Les fils de contact nécessaires sont solidarisés, par exemple à l'aide de colles conductrices, à chacune des deux électrodes se trouvant de part et d'autre du film sensible.

Pour assurer la protection mécanique et chimique de l'ensemble, une feuille de plastique telle que par exemple de PVDF non polarisé ou du PTFE recouvre l'ensemble de l'élément sensible.

Dans une telle configuration, un signal électrique sera enregistré pour tout effort exercé perpendiculairement ou longitudinalement à la feuille piézo-électrique.

L'élimination d'effets parasites à la détection, tels par exemple la création de signaux pyro-électriques ou encore la création de signaux dus à des mouvements du sujet observé peut être réalisée par tout moyen approprié, notamment en utilisant deux éléments sensibles par détecteur.

Dans cette configuration, un seul des éléments piézo-sensibles est exposé aux perturbations créées par le paramètre mesuré, alors que l'autre élément est exposé à toutes les autres stimulations communes. Par le raccordement parallèle des transducteurs ou par un traitement adéquat des données, tous les signaux communs aux deux éléments s'annuleront.

L'élément piézo-sensible peut enregister simultanément plusieurs des paramètres tels par exemple les rythmes cardiaques et respiratoires et/ou la pression artérielle et ce, soit en utilisant un seul transducteur dont les signaux sont analysés globalement et décomposés par l'unité de traitement électronique, soit encore en disposant sur la circonférence de la ceinture autant d'éléments piézo-sensibles qu'il y a de fonctions à observer et en traitant chacun des signaux détectés dans une unité individuelle.

Un capteur de température, tel par exemple un thermocouple ou une sonde résistive de platine en couche mince peut être intégré à la ceinture, afin d'enregistrer également la température du corps du sujet.

En alternative à l'utilisation de films plastiques piézo-électriques, l'élément sensible de la ceinture peut également être un film de caoutchouc piézo-sensible ou une jauge de contrainte ou encore une chambre déformable et un capteur de pression.

Il existe actuellement des caoutchoucs piézo-sensibles tels par exemple des silicones chargés de particules électriquement conductrices et dont la résistivité électrique est modifée considérablement lors de déformations mécaniques imposées à un élément de ce matériau.

En raccordant l'élément piézo-résistif à un pont de Wheastone excité par une tension continue, l'application d'une déformation de la ceinture se traduira par une variation de la tension de sortie du pont.

Les jauges de contrainte sont actuellement utilisées dans des applications aussi diverses que l'analyse de contraintes et de déformations dans des structures mécaniques ou de génie civil ou encore comme élément sensible dans des capteurs tels que par exemple les capteurs de force, de déformation ou de pression.

Les jauges sont en général déposées sur un support tel du verre ou des polymères comme par exemple des polyimides.

Il existe différents types de matériaux de jauge.

En gros, ils peuvent être regroupés en trois classes :

(1) jauges métalliques;

(2) jauges semi-conductrices;

(3) jauges métallo-céramiques (CERMET).

Les jauges métalliques sont de loin les plus courantes.

Le principe de fonctionnement des jauges est piézo-résistif, c'est-à-dire que toute contrainte mécanique imposée à la jauge se traduit par une déformation qui elle même se traduit par une variation de la résistance électrique nominale de la jauge.

Les jauges se caractérisent principalement par le facteur de jauge défini comme étant :

$$k = \frac{\Delta R}{R} \cdot \frac{1}{\epsilon} ,$$

où
R est la résistance nominale,
$\Delta R$, la variation de résistance et
$\epsilon$, la déformation relative

$$\frac{(\Delta L)}{(L)} .$$

Les jauges métalliques présentent en général un facteur de jauge de 2, les jauges CERMET de 15 et les jauges semi-conductrices de 80.

Ces dernières facilitent par conséquent la détection de variations de résistance, étant donné qu'à déformation égale, la variation de résistance est environ 40 fois plus importante.

Les jauges métalliques restent néanmoins les plus utilisées car elles sont les plus stables et les moins dépendantes de la température ambiante.

Les variations de résistance sont mesurées en général à l'aide d'un pont de Wheastone qui permet, lorsque ce dernier est alimenté par une tension continue, de mesurer la tension de déséquilibre du pont résultant de la variation des résistances constituant le pont.

Lorsqu'on utilise une chambre déformable comprenant un fluide tel par exemple de l'air et un capteur de pression, toute déformation subie par la ceinture se traduira par une variation de volume de la chambre déformable. Cette dernière engendrera une variation de la pression interne, qui à son tour sera détectée par le capteur de pression.

Une particularité supplémentaire de la présente invention réside dans l'utilisation connue en soi cependant de liens radio entre le sujet "surveillé" (animal ou humain) et le surveillant (éleveur ou soigneur) ou entre le sujet surveillé et une centrale de traitement de l'information ou une centrale d'alarme.

Sur un plan purement technique, il est important de prendre en compte le fait que l'invention présente les avantages suivants :

- ceinture active combinant à la fois une composante élastique et la transmission de l'effort mécanique
- annulation des composantes statiques (position du sujet ou de la ceinture, signaux à longues périodes) par une correction automatique du décalage (offset)
- analyse par une seule et même ceinture de plusieurs paramètres permettant une corrélation des signaux relevés.

De par sa conception la ceinture de l'invention du fait qu'elle travaille longitudinalement offre de plus l'avantage que la position de l'élément sensible par rapport à la zone surveillée est relativement peu importante.

Ainsi une contraction utérine peut être mesurée par une ceinture instrumentée englobant la partie ventrale du sujet mais dont l'élément sensible est situé sur le dos du sujet.

De même la pulsation cardiaque peut être en complément détectée par une ceinture instrumentée fixée dans la zone cardiaque mais dont l'élément sensible n'est pas situé en face du coeur.

Cet avantage apporte par rapport aux solutions existantes des avantages pratiques évidents :

- installation et mise en place de l'appareil non délicate et pouvant être exécutée par du personnel non qualifié,
- possibilité d'effectuer sans inconvénients des mesures ambulatoires.

## DESCRIPTION DES FIGURES

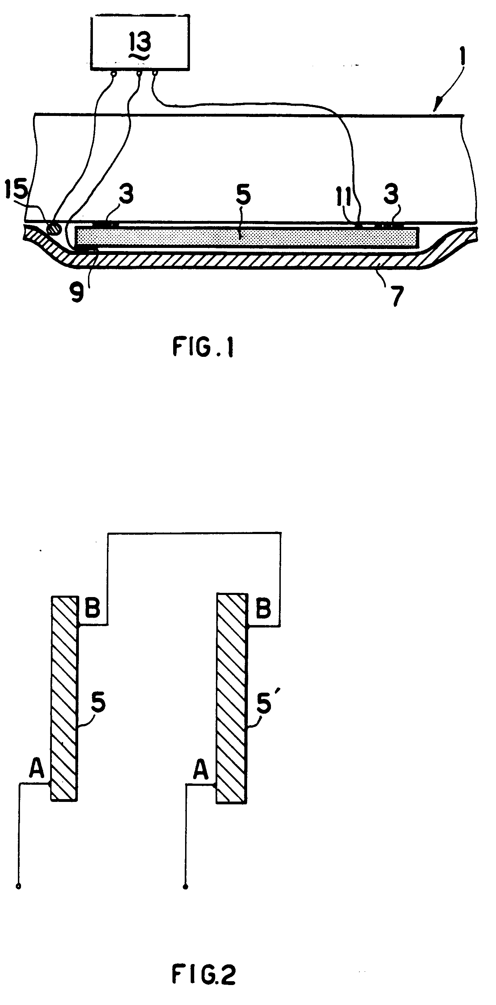

Dans les dessins annexés, la figure 1 représente une vue schématique en coupe d'une ceinture de surveillance.

Cette ceinture 1 qui entoure les flancs ou le thorax de l'animal ou de l'être humain comporte des moyens de fixations adéquats 3 pour un élément piézo-sensible 5, recouvert de préférence, ainsi qu'il a été indiqué ci-dessus, d'un film protecteur 7.

Des contacts 9 et 11 sont reliés par fils à une unité de traitement électronique schématisée par un bloc 13.

On a représenté en plus, à titre d'illustration, une sonde de température 15 reliée également au bloc 13.

La figure 2 représente le schéma d'un raccordement électrique compensateur en parallèle de deux éléments piézo-électriques. Deux éléments piézo-sensibles 5 et 5' sont montés en parallèle comme indiqué. L'un des élements est disposé de manière qu'il soit nécessairement sensible aux paramètres observés, tandis que l'autre est disposé de manière à ne pas être soumis à ceux-ci. Tous les signaux parasites ne résultant pas des paramètres observés (par exemple frottement de l'animal sur sa stalle) seront donc perçus par les éléments 5 et 5' et s'annuleront. En revanche, les paramètres observés, perçus nécessairement par un seul des deux éléments donneront un signal utile.

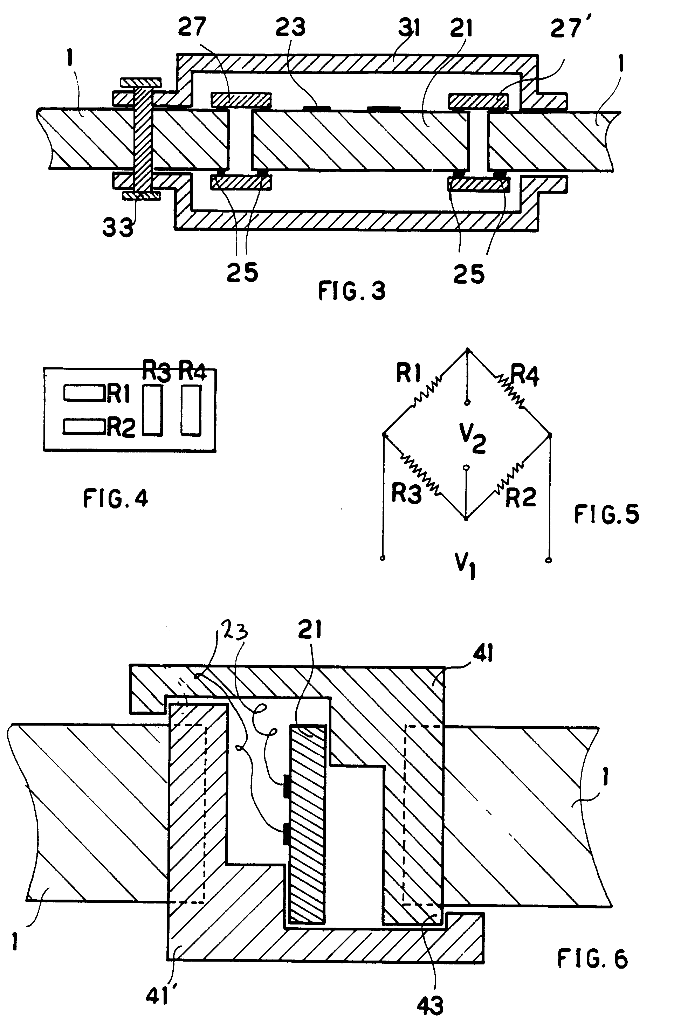

La figure 3 représente une vue schématique de la ceinture de surveillance comprenant comme élément sensible des jauges de contrainte.

Dans ce cas, la ceinture 1 est constituée d'une matière peu ou non déformable (par exemple un textile) et d'un élément déformable 21 sur lequel sont disposés les piézo-résistances 23 et qui est attachée rigidement à la ceinture par un ancrage ou un collage 25 et deux pièces d'attache 27 et 27'.

L'élément déformable 21 pourrait être par exemple un élastomère, ou encore une mince feuille métallique.

Les piézo-éléments ($R_1$ à $R_4$) sont disposés comme indiqué à la figure 4 afin de maximaliser la variation de résistance et de minimaliser les effets parasites tels que par exemple une variation de température.

Les piézo-éléments sont raccordés en pont de Wheastone comme indiqué à la figure 5, avec l'excitation $V_1$ et la sortie $V_2$.

Afin d'éviter une déformation excessive des piézo-résistances, la zone déformable de la ceinture est munie de part et d'autre d'un couvercle protecteur métallique 31 maintenu par un ancrage 33. Ceci empêche :

(1) une déformation excessive par déformation ou stimulation perpendiculaire,

(2) une déformation longitudinale excessive.

Cette dernière fonction est réalisée par exemple de la manière suivante.

Le couvercle 31 est ancré à la ceinture d'un seul côté de l'élément sensible par l'ancrage 33. De l'autre côté, le couvercle peut glisser sur cette dernière comme représenté. La pièce d'attache 27' qui relie l'élément déformable à la ceinture servira (du côté non ancré) de butée d'arrêt à la déformation longitudinale.

La figure 6 représente une autre configuration de ceinture munie de jauges de contrainte.

Dans ce cas, l'élément déformable 21 qui supporte les jauges de contrainte 23 est disposé de manière à ce que les déformations longitudinales soient converties en déformations transversales dans l'élément déformable.

Cette configuration permet d'utiliser un support déformable relativement rigide, tout en assurant une déformation relative adéquate pour la mesure.

Comme indiqué sur la figure 6, la déformation longitudinale de la ceinture l est transmise à l'élément déformable 21 par l'intermédiaire de deux armatures rigides 41 et 41' fixées rigidement à chaque extrémité de la ceinture 1 et exerçant un effort de flexion sur l'élément déformable 21.

Les armatures de déformation sont munies en outre d'un bras 43 servant de butée à l'autre armature et empêchant de la sorte les extensions excessives lors de la mise en place ou du stockage de l'appareil.

La forme d'exécution de la figure 3 est donc prévue essentiellement pour travailler en déformation longitudinale, celle de la figure 6 pour travailler essentiellement en déformation transversale.

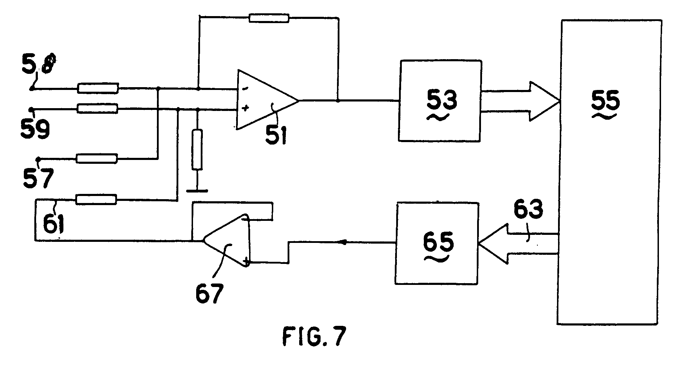

A titre d'exemple, on décrit ci-dessous, à l'appui de la figure 7, une forme d'exécution d'un circuit de traitement électronique selon l'invention.

Selon l'invention, le circuit de traitement électronique comporte au moins un moyen d'amplification du signal, un moyen de différenciation du signal capté parmi les signaux parasites et un moyen de correction automatique de la ligne de base des signaux.

En référence à cette figure, le circuit de traitement électronique comporte essentiellement un amplificateur des signaux d'entrée monté en amplificateur différentiel 51 et éventuellement un amplificateur à gain élevé monté en aval de celui-ci, dont les signaux sont envoyés dans un convertisseur analogique/digital 53 pour être traités par une unité de traitement 55 tel qu'un micro-processeur. Les diverses sorties (non représentées) du micro-processeur sont utilisées pour la régulation de certaines fonctions annexes et pour la commande des organes d'alarme ..etc. Par fonctions annexes, on entend la correction automatique de la ligne de base, la régulation des tensions d'alimentation et autres.

Le conduit 58 du signal de la sonde montée en parallèle est connecté à une tension de référence 57 avant d'être relié à la première entrée de l'amplificateur différentiel 51 et le conduit 59 du signal de la sonde de mesure est connecté à la tension de correction de la ligne de base 61 avant d'être relié à la deuxième entrée de l'amplificateur différentiel 51.

Un logiciel adéquat permet la réalisation d'une série de fonctions énumérées à titre non limitatif ci-dessous:

- contrôle initial de la contrainte statique dans la ceinture afin de valider son installation sur le sujet à examiner,

- lecture de signaux consécutifs,

- filtrage des signaux mesurés,

- calcul de la nouvelle valeur du contrôle de la ligne de base et ajustement automatique de la position de celle-ci,

- identification de contractions abdominales et/ou utérines,
- analyse des paramètres des contractions,
- émission d'une alarme quand un des phénomènes suivants est reconnus :
  - premières contractions utérines,
  - apparition des premières contractions abdominales,
  - apparition des premières contractions expulsives,
  - non-apparition de l'un ou l'autre de ces phénomènes après un certain laps de temps,
- tests de fonctionnement du capteur et émission de signaux en cas de défaut.

La sortie 63 du microprocesseur 55 destinée à la correction de la ligne de base est raccordée à un convertisseur digital/analogique 65 qui est relié (61), éventuellement via un amplificateur 67, à l'amplificateur différentiel 51.

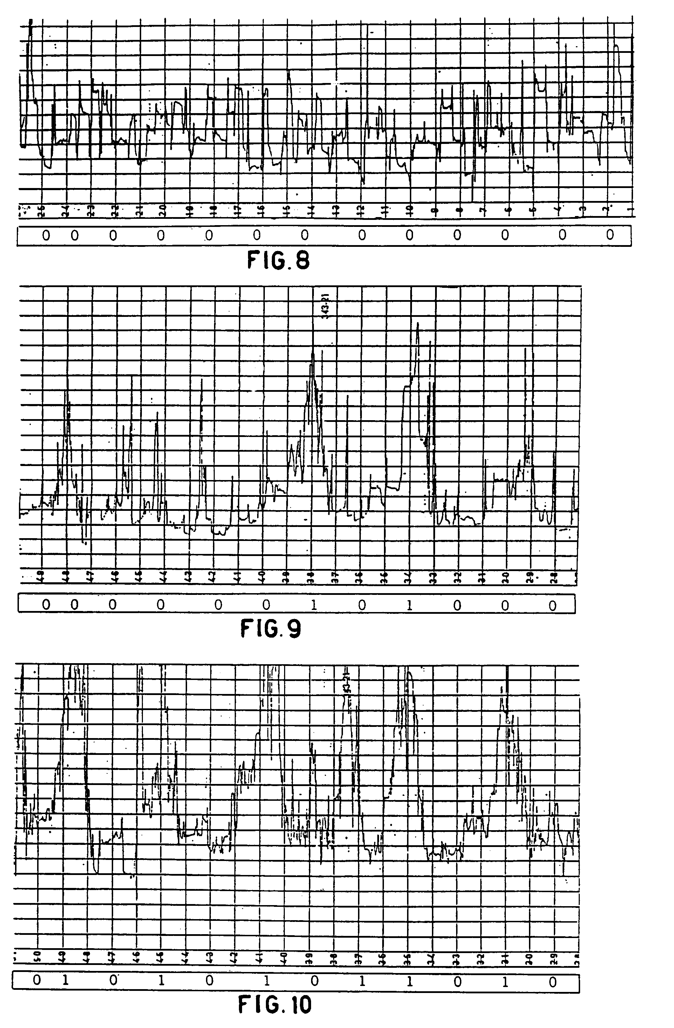

Les figures 8, 9 et 10 représentent une séquence du paramètre analysé, obtenue en faisant une représentation en série des phénomènes détectés. Un registre est incrémenté d'une valeur "1" ou "0" suivant qu'un phénomène a été détecté ou pas. Un étude comparative et différentielle permet d'évaluer l'évolution du phénomène.

A la figure 8, on ne détecte pas encore d'effet se distinguant dans le graphique.

A la figure 9, on constate un début d'activité intense et à la figure 10, l'activité est très intense.

De nombreuses autres formes d'exécution sont bien entendu possibles dans le cadre de l'invention.

Dans les dessins des repères de référence identiques ont été utilisés pour des éléments constitutifs à fonction similaire, mais non nécessairement identiques. Le choix des matériaux mis en oeuvre sera bien entendu chaque fois adapté aux efforts à supporter et aux fonctions à exercer.

A titre d'exemples, on décrit deux configurations possibles d'utilisation de la ceinture de surveillance.

Exemple 1
‒ ‒ ‒ ‒ ‒ ‒

La ceinture telle que représentée sur les figures 1, 3 et 6 est munie d'une unité de traitement électronique autonome alimentée par piles ou batterie. L'unité permet de détecter les premières contractions utérines et/ou abdominales et à cet instant, un signal d'alarme est envoyé par ondes radio depuis l'unité de traitement vers un récepteur portatif porté en permanence par l'éleveur ou le soigneur.

Exemple 2
‒ ‒ ‒ ‒ ‒ ‒

La ceinture est munie d'une unité de prétraitement (amplificateur) qui envoie par ondes radio des signaux reçus vers une unité de traitement centrale située non loin de l'être surveillé et pouvant traiter les signaux provenant de plusieurs ceintures de surveillance.

Dès que l'unité centrale enregistre une observation critique, un signal est envoyé soit vers une alarme centrale, soit vers des récepteurs radio portés par le soigneur et son personnel.

**Revendications**

1. Dispositif de surveillance de la parturition pour les animaux et êtres humains comportant une ceinture de surveillance comportant au moins un élément sensible (5) qui permet de détecter l'activité musculaire abdominale et en particulier les contractions utérines et abdominales et qui est relié à un circuit électronique (13) de traitement qui permet de suivre l'évolution an amplitude et en fréquence des contractions utérines et abdominales et permet notamment une correction de la ligne de base, caractérisé en ce que le circuit électronique (13) comprend, pour ce faire, un amplificateur des signaux d'entrée, monté en amplificateur différentiel (51), un conduit (58) du signal des éléments sensibles et une tension de référence (57) étant connectés à une première entrée de l'amplificateur différentiel (51) et un autre conduit (59) du signal des éléments sensibles et une tension de correction de la ligne de base (61) étant connectés à la deuxième entrée de l'amplificateur différentiel (51), la tension de correction de la ligne de base étant fournie par un microprocesseur (55) après analyse par celui-ci et conversion digitale/analogique (65) du signal de la sortie de l'amplificateur différentiel (51), ledit microprocesseur faisant également une analyse de l'évolution de l'activité musculaire pour déclencher une alarme ou un système de prévention.

2. Dispositif selon la revendication 1 caractérisé en ce que ledit élément sensible (5) agit dans le sens longitudinal de déformation de ladite ceinture.

3. Dispositif selon l'une quelconque des revendications 1 ou 2 caractérisé en ce qu'il est relié sans fil, notamment par radio, par infrarouge ou par ultrasons à la centrale de traitement de l'information ou à une centrale d'alarme ou à un récepteur portatif.

**4.** Dispositif selon l'une quelconque des revendications précédentes caractérisé en ce qu'il comporte différents indicateurs d'alarme permettant de déceler une défectuosité du dispositif et/ou l'état d'avancement de la parturition.

**5.** Dispositif selon l'une quelconque des revendications précédentes caractérisé en ce qu'il comporte des moyens permettant de déclencher l'alarme en prenant en compte la variation individuelle de la ligne de base pour le sujet qui en est équipé.

**6.** Dispositif selon la revendication 5 caractérisé en ce que ladite ceinture comporte des éléments sensibles (5) qui permettent d'enregistrer au moins l'un des paramètres suivants :
- température du corps
- rythme respiratoire
- rythme cardiaque
- pression artérielle.

**7.** Dispositif selon l'une quelconque des revendications précédentes caractérisé en ce que l'élément sensible (5) de la ceinture (1) de surveillance est constitué par une feuille plastique piézo-électrique, par exemple constituée de PVDF (difluorure de polyéthylène) ou de composites comprenant des particules céramiques piézo-électriques enrobées dans une matrice polymérique; ou bien par un film de caoutchouc piézo-sensible ou par au moins une jauge de contrainte ou encore par une chambre déformable comprenant un fluide tel que de l'air et un capteur de pression, ladite ceinture de surveillance comportant de préférence deux éléments sensibles (5,5') par détecteur montés de telle sorte qu'un seul des éléments sensibles est exposé aux perturbations créées par le ou les paramètres observés, alors que l'autre élément est exposé à toutes les autres stimulations communes, les deux éléments sensibles étant raccordés de sorte que tous les signaux communs aux deux éléments s'annulent.

**8.** Utilisation du dispositif selon l'une quelconque des revendications 1 à 7 pour la détection, la surveillance et/ou l'enregistrement de l'activité musculaire, notamment les contractions abdominales et utérines, en médecine humaine ou vétérinaire ou à l'élevage.

**Claims**

**1.** Parturition monitoring device for animals and human beings comprising a monitoring belt which comprises at least one sensing element (5) which enables abdominal muscular activity and in particular, uterine and abdominal contractions to be detected and which is connected to an electronic treatment circuit (13) which enables to follow the changes in the amplitude and in the frequency of the uterine and abdominal contractions and which enables in particular a base line correction, characterized in that the electronic circuit (13) comprises therefore an amplifier for input signals, assembled as differential amplifier (51), a sensing element signal line (58) and a reference voltage (57) being connected to the first input of the differential amplifier (51) and a second sensing element signal line (59) and a base line correction voltage (61) being connected to the second input of the differential amplifier (51), the base line correction voltage being supplied by a microprocessor (55) after analysis and analogue/digital conversion (65) of the output signal of the differential amplifier (51); said microprocessor also analysing the changes in the muscular activity enabling an alarm or safety system to be triggered.

**2.** Device according to claim 1 characterized in that said sensing element (5) is acting in the longitudinal direction of deformation of said belt.

**3.** Device according to any one of the claims 1 or 2, characterized in that it is connected, wireless, in particular by radio, by infrared or by ultrasonics to the data processing station or to an alarm station or to a portable receiver.

**4.** Device according to any one of the preceding claims, characterized in that it comprises different alarm indicators which enable a defect of the device and/or the state of progress of parturition to be detected.

**5.** Device according to any one of the preceding claims, characterized in that it comprises means which enable the alarm to be triggered by taking into account the individual base line variation for the subject fitted therewith.

**6.** Device according to claim 5, characterized in that said belt comprises sensing elements (5) which enable at least one of the following parameters to be recorded:
- body temperature,
- respiratory cycle,
- cardiac cycle,
- arterial pressure.

**7.** Device according to any one of the preceding claims characterized in that the sensing element (5) of the monitoring belt (1) consists of a piezoelectric plastic sheet, for example consisting of PVDF (polyvinylidene fluoride) or of compounds containing coated piezoelectric ceramic particles in a polymer matrix; or of a piezosensing rubber film or of at least one strain gauge or of a deformable chamber containing a fluid such as air and a pressure sensor, said monitoring belt comprising preferably two sensing elements (5, 5') per detector, mounted so that only one of the sensing elements is exposed to disturbances produced by the parameter(s) monitored, whereas the other element is exposed to all other common stimulations, the two sensing elements being connected so that all signals common to the two elements cancel themselves out.

8. Use of the device according to any one of claims 1 to 7 for the detection, the monitoring and/or the recording of muscular activity, in particular uterine and abdominal contractions, in human and veterinary medicine or in the breeding.

**Patentansprüche**

1. Vorrichtung zur Überwachung des Geburtsvorgangs bei Tieren und Menschen, aus einem Überwachungsgürtel, der mindestens ein Sensorelement (5) aufweist, mit dem die Bauchmuskelaktivität und insbesondere die Gebärmutter- und Bauchkontraktionen festgestellt werden können, und das mit einer elektronischen Verarbeitungsschaltung (13) verbunden ist, mit der die Entwicklung der Gebärmutter- und Bauchkontraktionen hinsichtlich der Amplitude und der Frequenz verfolgt werden kann, und mit der insbesondere eine Korrektur der Grundlinie vorgenommen werden kann, **dadurch gekennzeichnet,** daß die elektronische Schaltung (13) dazu einen Verstarker für die Eingangssignale enthält, der als Differentialverstärker (51) geschaltet ist, bei dem ein Signal (58) der Sensorelemente und eine Bezugsspannung (57) auf einen ersten Eingang des Differentialverstärkers (51) gegeben werden, und ein weiteres Signal (59) der Sensorelemente und eine Spannung (61) zur Korrektur der Grundlinie auf den zweiten Eingang des Differentialverstärkers (51) gegeben werden, wobei die Spannung zur Korrektur der Grundlinie von einem Mikroprozessor (55) geliefert wird, nach Analyse und digitaler/analoger Umwandlung (65) des Ausgangssignals des Differentialverstärkers (51) durch den besagten Mikroprozessor, der ebenfalls eine Analyse der Entwicklung der Muskelaktivität macht, um einen Alarm oder ein Vorbeugungssystem auszulösen.

2. Vorrichtung gemäß Anspruch 1, **dadurch gekennzeichnet, daß** das besagte Sensorelement (5) in der Längsrichtung der Verformung des besagten Gürtels wirkt.

3. Vorrichtung gemäß irgendeinem der Ansprüche 1 oder 2, **dadurch gekennzeichnet, daß** sie drahtlos, insbesondere über Funk-, Infrarot- oder Ultraschallwellen mit der Informationsverarbeitungszentrale oder einer Alarmzentrale oder einem tragbaren Empfänger verbunden ist.

4. Vorrichtung gemäß irgendeinem der vorhergehenden Ansprüche, **dadurch gekennzeichnet, daß** sie verschiedene Alarmindikatoren aufweist, mit denen eine Schadhaftigkeit der Vorrichtung und/oder der Fortgang des Geburtsvorgangs festgestellt werden können.

5. Vorrichtung gemäß irgendeinem der vorhergehenden Ansprüche, **dadurch gekennzeichnet, daß** sie Mittel aufweist, die ermöglichen, den Alarm auszulösen unter Berücksichtigung der individuellen Variation der Grundlinie für den Patienten, der mit dieser Vorrichtung ausgerüstet ist.

6. Vorrichtung gemäß Anspruch 5, **dadurch gekennzeichnet, daß** der besagte Gürtel Sensorelemente (5) aufweist, mit denen mindestens einer der folgenden Parameter aufgezeichnet werden kann :
   - Körpertemperatur
   - Atmungsrhythmus
   - Herzrhythmus
   - Blutdruck.

7. Vorrichtung gemäß irgendeinem der vorhergehenden Ansprüche, **dadurch gekennzeichnet, daß** das Sensorelement (5) des Überwachungsgürtels (1) gebildet wird von einer piezoelektrischen Plastikfolie, beispielsweise aus PVDF (Polyvinyldifluorid), oder aus Verbundmaterialien, die piezoelektrische keramische Partikel enthalten, die in eine polymere Matrix eingebettet sind; oder von einem piezoempfindlichen Gummifilm, oder von mindestens einem Dehnungsmeßstreifen, oder von einer verformbaren Kammer, die ein Fluid, wie Luft, und einen Drucksensor enthält, wobei der besagte Überwachungsgürtel vorzugsweise zwei Sensorelemente (5,5') pro Meßstelle aufweist, die so angebracht sind, daß eines der Sensorelemente den Störungen ausgesetzt ist, die von dem oder den beobachteten Parametern hervorgerufen werden, während das andere Sensorelement allen anderen gemeinsamen Stimulationen ausgesetzt ist, wobei die zwei Sensorelemente so angeschlossen sind, daß alle für sie gemeinsamen Signale sich gegenseitig aufheben.

8. Verwendung der gemäß irgendeinem der Ansprüche 1 bis 7 beanspruchten Vorrichtung zum Nachweis, zur Überwachung, und/oder zur Aufzeichnung der Muskelaktivität, insbesondere der Bauch- und Gebärmutterkontraktionen in der Human- oder Veterinärmedizin oder bei der Tierzucht.

FIG.1

FIG.2

EP 0 277 941 B1

FIG. 3

FIG. 4

FIG. 5

FIG. 6

FIG. 7

FIG.8

FIG.9

FIG.10